# EUROPEAN PATENT APPLICATION

(11) **EP 1 600 062 A1**
(43) Date of publication of application: **30.11.2005**
(21) Application number: 04012289.7
(22) Date of filing: 25.05.2004
(51) Int. Cl.: A23L 1/30, A23L 1/307, A23L 1/308, A23L 1/054, A23L 1/09, A23L 1/236, A23K 1/165, A61K 31/733, A61K 31/7016, A61K 31/702, A61K 31/715, A61K 31/716

(54) **Oral and/or topical compositions comprising prebiotics and sterols**

(71) Applicant: Cognis IP Management GmbH, 40589 Düsseldorf (DE)
(72) Inventor: Bell, Doris, Dr., 40627 Düsseldorf (DE); Rull Prous, Santiago, 08034 Barcelona (ES); Fabry, Bernd, Dr., 41352 Korschenbroich (DE)

(57) **Abstract**

Suggested are new advantageous oral and/or topical compositions, comprising
(a) prebiotics and
(b) sterols or their esters.

## Description

### Field of the invention

The present invention is related to the area of alimentation and concerns oral and/or topical compositions comprising sterols or their esters and prebiotics, dietary supplements and food compositions comprising sterols or their esters and prebiotics, and the use of mixtures comprising sterols or their esters and prebiotics for improving the stimulation of the growth of healthy bacteria.

### Background of the invention

Probiotics contain live bacteria and represent an important part of the complex world of foods that are good for health. Its the bacteria and the metabolites which they produce that give these products their health promoting properties. The best known example of a probiotic is yoghurt. The experimental data for yoghurt is still not as conclusive as one would like, however, human studies related to the consumption of dietary milk products show increased milk digestibility, quicker recovery from certain types of diarrhoea, enhanced immune function, relation in certain cancers, and possible lowering of blood cholesterol levels.

Bacteria found in products like yoghurt, kefir or fermented vegetables usually aren't found in the human intestine. In fact, the intestinal environment is often a hostile one for these foreign bacteria. Because of this, bacteria eaten in probiotic products don't colonise the intestine but are flushed through and eliminated from the body.

The bacteria living in the intestine make up a very large and very diverse population. The numbers of each kind of bacteria change depending on age, diet, health status, and use of drugs and supplements. The effects are linked to the ability of the bacteria to adhere to the intestinal wall and use the semi-digested food that it passing through the intestines. It is not surprising to found that the bacterial population in the intestines of vegetarians is much different compared to that of meat eaters. Because some bacteria have specific nutrient requirements it has been proposed that adding these particular foods or nutrients to the diet could be a way of increasing the numbers of specific bacteria. Such additives are called "prebiotics". Thus, to be effective, prebiotics must escape digestion in the upper gastrointestinal tract and be used by a limited number of the micro-organisms comprising the colonic microflora. Typical examples for well-known prebiotics are oligosaccharides, e.g. in 1995 Gibson et al found that oligofructose and inulin, when fed to humans, selectively stimulated the growth of bifido-bacteria without influencing the numbers of lactobacillus. Since prebiotics mainly stimulate the growth of bifidobacteria, for which reason the also are referred to as bifidogenetic factors.

Although various types of prebiotics are known from the literature and can be found in the market there is still an increasing need for more active alternatives or additives which support the various activities of existing products in synergistic manner. Therefore, the object of the present invention has been to provide a new system of prebiotic compounds which shows a synergistic stimulation of the growth of healthy bacteria, preferably bifido and lactic bacteria both.

### Detailed description of the invention

The present invention refers to oral and/or topical compositions, comprising
(a) prebiotics and
(b) sterols or their esters.

Surprisingly it has been observed that mixtures of said sterols or sterol esters and prebiotics show a synergistic behaviour with respect to stimulation of growth of bacteria selected from the group consisting of *Bifidobacterium breve, Bifidobacterium infantis*, *Bifidobacterium longum* and *Bifidobacterium adolescentis* on one hand, and *Lactobacillus bulgaricus, Lactobacillus acidophilus*, *Lactobacillus casei, Lactobacillus plantarum*, *Streptococcus faecium, and Streptococcus thermophilus* on the other.

### Prebiotics

Prebiotics are defined as non-digestible food ingredients that may beneficially affect the host be selectively stimulating the growth and/or the activity of a limited number of bacteria in the colon. The following describes the various oligosaccharides which can be taken into account as suitable prebiotics (component a) :

### • Fructooligosaccharides

Fructooligosaccharides or FOS typically refer to short-chain oligosaccharides comprised of D-fructose and D-glucose, containing from three to five monosaccharide units. FOS, also called neosugar and short-chain FOS, are produced on a commercial scale from sucrose using a fungal fructosyltransferase enzyme. FOS are resistant to digestion in the upper gastrointestinal tract. They act to stimulate the growth of *Bifidobacterium* species in the large intestine. FOS are marketed in the United States in combination with probiotic bacteria and in some functional food products.

### • Inulins

Inulins refer to a group of naturally-occurring fructose-containing oligosaccharides. Inulins belong to a class of carbohydrates known as fructans. They are derived from the roots of chicory *(Cichorium intybus)* and Jerusalem artichokes. Inulins are mainly comprised of fructose units and typically have a terminal glucose. The bond between fructose units in inulins is a beta-(2-1) glycosidic linkage. The average degree of polymerisation of inulins marketed as nutritional supplements is 10 to 12. Inulins stimulate the growth of *Bifidobacterium* species in the large intestine.

### • Isomaltooligosaccharides

Isomaltooligosaccharides comprise a mixture of alpha-D-linked glucose oligomers, including isomaltose, panose, isomaltotetraose, isomaltopentaose, nigerose, kojibiose, isopanose and higher branched oligosaccharides. Isomaltooligosaccharides are produced by various enzymatic processes. They act to stimulate the growth of *Bifidobacterium* species and *Lactobacillus* species in the large intestine. Isomalto oligosaccharides are marketed in Japan as dietary supplements and in functional foods. They are being developed in the United States for similar uses.

### • Lactilol

Lactilol is a disaccharide analogue of lactulose. Its pharmaceutical use is in the treatment of constipation and hepatic encephalopathy. Lactilol is also used in Japan as a prebiotic. It is resistant to digestion in the upper gastrointestinal tract and it is fermented by a limited number of colonic bacteria, resulting in an increase in the biomass of bifidobacteria and lactobacilli in the colon. Lactilol is known chemically as 4-0-(beta-D-galactopyranosyl)-D-glucitol. Lactilol is not approved for the treatment of hepatic encephalopathy or constipation in the U.S., and its use as a prebiotic is considered experimental. Lactilol is used in Europe as a food sweetener.

### • Lactosucrose

Lactosucrose is a trisaccharide comprised of D-galactose, D-glucose and D-fructose. Lactosucrose is produced enzymatically by the enzymatic transfer of the galactosyl residue in lactose to sucrose. Lactosucrose is resistant to digestion in the stomach and small intestine. It is selectively utilized by intestinal *Bifidobacterium* species resulting in significant induction of growth of these bacteria in the colon. Therefore, under physiological conditions, lactosucrose acts on the intestinal microflora as a growth factor for *Bifidobacterium* species. Lactosucrose is also known as 4G-beta-D-galactosylsucrose. It is widely used in Japan as a dietary supplement and in functional foods, including yoghurt. Lactosucrose is being developed in the United States for similar uses.

### • Lactulose

Lactulose is a semi-synthetic disaccharide comprised of the sugars D-lactose and D-fructose. The sugars are joined by a beta-glycosidic linkage, making it resistant to hydrolysis by human digestive enzymes. Lactulose is, however, fermented by a limited number of colonic bacteria. This can lead to changes in the colonic ecosystem in favour of bacteria, such as lactobacilli and bifidobacteria, which may confer some health benefits. Lactulose is a prescription drug in the United States for the treatment of constipation and hepatic encephalopathy. It is marketed in Japan for use as a dietary supplement and in functional foods. Its use in the United States as a prebiotic substance is still experimental.

### • Pyrodextrins

Pyrodextrins comprise a mixture of glucose-containing oligosaccharides that is derived from the hydrolysis of starch. Pyrodextrins have been found to promote the proliferation of *Bifidobacterium* species in the large intestine. They are resistant to digestion in the upper gastrointestinal tract. Pyrodextrins are being developed for the nutritional supplement market place.

### • Soy oligosaccharides

Soy oligosaccharides refer to oligosaccharides found in soybeans and also in other beans and peas. The two principal soy oligosaccharides are the trisaccharide raffinose and the tetrasaccharide stachyose. Raffinose comprises one molecule each of D-galactose, D-glucose and D-fructose. Stachyose consists of two molecules of D-galactose, one molecule of D-glucose and one molecule of D-fructose. Soy oligosaccharides act to stimulate the growth of *Bifidobacterium* species in the large intestine. They are marketed in Japan as dietary supplements and in functional foods. They are being developed in the United States for similar uses.

### • Transgalactooligosaccharides

Transgalactooligosaccharides (TOS) are a mixture of oligosaccharides consisting of D-glucose and D-galactose. TOS are produced from D-lactose via the action of the enzyme beta-galactosidase obtained from *Aspergillus oryzae.* TOS are resistant to digestion in the upper gastrointestinal tract and stimulate the growth of bifidobacteria in the large intestine. TOS are marketed in Japan and Europe as dietary supplements and are used in functional foods. They are being developed for similar use in the United States.

### • Xylooligosaccharides

Xylooligosaccharides are comprised of oligosaccharides containing beta (1→4) linked xylose residues. The degree of polymerisation of xylooligosaccharides is from two to four. Xylo oligosaccharides are obtained by enzymatic hydrolysis of the polysaccharide xylan. They are marketed in Japan as prebiotics and are being developed for similar use in the United States.

### • Beta Glucans

Suitable beta-glucans include those originating from plants including cereals such as oats and barley, fungi, yeast, and bacteria. In addition, microbial cell wall preparations and whole cells rich in beta glucans are also suitable sources for beta glucan preparations useful for the present invention. Monomer residues in glucans can have 1-3 and 1-4, or 1-3 and 1-6 linkages (that is the monomer units are joined through 1,3, 1,4 or 1,6 bonds) and the percent of each type can vary. Preferably, beta glucans derived from yeast, particularly from *Saccharomyces,* preferably *Saccharomyces cerevisiae*, are used for the present invention. It will be appreciated, however, that other beta glucans would also be suitable.

### Sterols and sterol esters

Sterols - also called sterins - represent steroids showing a single hydroxyl group linked to the C-3. In addition sterols, which consist of 27 to 30 carbon atoms, may show a double bond, preferably in 5/6 position. The hydrogenation of the double bond ("hardening") leads to sterols which are usually called stanols. The figure below shows the structure of the best known member of the sterol family, cholesterol, which belongs to the group of zoosterols.

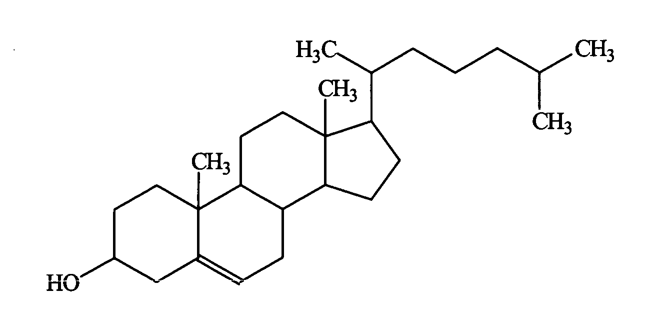

Due to their superior physiological activity, the plant sterols, so-called phytosterols, like ergosterol, stigmasterol, and especially sitosterol and its hydrogenation product sitastanol, are the preferred species. In addition instead of the sterols or stanols their esters with saturated or unsaturated fatty acids having 6 to 26 carbon atoms and up to 6 double bonds can be used. Typical examples are the esters of β-sitosterol or β-sitostanol with capric acid, caprylic acid, 2-ethylhexanoic acid, caprinic acid, lauric acid, isotridecylic acid, myristic acid, palmitic acid, palmoleic acid, stearic acid, isostearic acid, oleic acid, elaidinic acid, petroselinic acid, linolic acid, linoleic acid, elaeostearic acid, arachidonic acid, gadoleinic acid, behenic acid and erucic acid.

### Oral and/or topical compositions

The oral and/or topical compositions according to the present invention may comprise the prebiotics and the sterols or their esters in a weight ratio of 99 to 1 to 50 : 50 and more particularly 95 : 10 to 75 : 25. The highest synergistic effects, however, are observed at ratios of 92 : 8 to 80 : 20. In general, the compositions can be used in a concentration of up to about 10, particularly 0.5 to 8 and more particularly 1 to 2 % b.w. - calculated on the probiotic micro-organisms being present in the final food composition. One percent, however, has been found to be particularly suitable.

In a special embodiment of the present invention said compositions are macro- or micro-encapsulated. "Microcapsules" are understood to be spherical aggregates with a diameter of about 0.1 to about 5 mm which contain at least one solid or liquid core surrounded by at least one continuous membrane. More precisely, they are finely dispersed liquid or solid phases coated with film-forming polymers, in the production of which the polymers are deposited onto the material to be encapsulated after emulsification and coacervation or interfacial polymerization. In another process, liquid active principles are absorbed in a matrix ("micro-sponge") and, as microparticles, may be additionally coated with film-forming polymers. The microscopically small capsules, also known as nanocapsules, can be dried in the same way as powders. Besides single-core microcapsules, there are also multiple-core aggregates, also known as microspheres, which contain two or more cores distributed in the continuous membrane material. In addition, single-core or multiple-core microcapsules may be surrounded by an additional second, third etc. membrane. The membrane may consist of natural, semisynthetic or synthetic materials. Natural membrane materials are, for example, gum arable, agar agar, agarose, maltodextrins, alginic acid and salts thereof, for example sodium or calcium alginate, fats and fatty acids, cetyl alcohol, collagen, chitosan, lecithins, gelatin, albumin, shellac, polysaccharides, such as starch or dextran, polypeptides, protein hydrolyzates, sucrose and waxes. Semisynthetic membrane materials are inter alia chemically modified celluloses, more particularly cellulose esters and ethers, for example cellulose acetate, ethyl cellulose, hydroxypropyl cellulose, hydroxypropyl methyl cellulose and carboxymethyl cellulose, and starch derivatives, more particularly starch ethers and esters. Synthetic membrane materials are, for example, polymers, such as polyacrylates, polyamides, polyvinyl alcohol or polyvinyl pyrrolidone. Examples of known microcapsules are the following commercial products (the membrane material is shown in brackets) *Hallerest Microcapsules* (gelatin, gum arabic), *Coletica Thalaspheres* (maritime collagen), *Lipotec Millicapseln* (alginic acid, agar agar), *Induchem Unispheres* (lactose, microcrystalline cellulose, hydroxypropylmethyl cellulose), *Unicerin C30* (lactose, microcrystalline cellulose, hydroxypropylmethyl cellulose), *Kobo Glycospheres* (modified starch, fatty acid esters, phospholipids), *Softspheres* (modified agar agar), *Kuhs Probiol Nanospheres* (phospholipids) and *Primaspheres* or *Primasponges* (chitosan, anionic polymers).

### Food compositions

A further object of the present invention relates to food compositions, comprising
(a) prebiotics and
(b) sterols or their esters.

The compositions may further comprise certain plant extracts, like extracts of *Camellia sinensis* (Green tea) or *Olea europensis* (Olive tree) which are rich in actives like polyphenols, oleuropein and hydroxtyrosol.

### Industrial application

A final object of the present invention is related to the use of mixtures, comprising
(a) prebiotics and
(b) sterols or their esters
for stimulating the growth of healthy bacteria, for example in the stomach (if applied oral) or on skin (if administered topical).

### Examples

### Examples 1 to 10, Comparative Examples C1 to C18

The stimulation of growth of micro-organisms has been studied by enumerating *bifidobacterium* and *lactobacilli* in vitro in the presence of various test substances. More specifically, aliquots (1 mL) of human faecal homogenates (10 g per 100 mL diluent) were added to diluted WC broth (diluted 50:50 with 0.05M phosphate buffer) to which were added the test mixtures and a lactobacillus or bifidobacterium strain. For each of the combinations, parallel tubes were prepared with one set being inoculated with *Bifidobacterium spp* or *Lactobacillus spp.* All mixtures were then incubated for up to 24 hours and bacterial numbers enumerated. The results are presented in Tables 1 and 2:

**Table 1**

| **Effect of 1 % prebiotic, sterols and prebiotic/sterol mixture on** ***Bifidobacterium*** | | | | | | | | | | | | | | |
|---|---|---|---|---|---|---|---|---|---|---|---|---|---|---|
| | **0** | **C1** | **C2** | **C3** | **C4** | **C5** | **C6** | **C7** | **C8** | **1** | **2** | **3** | **4** | **5** |
| Inulin | - | 1.0 | - | - | - | - | - | - | - | 0.8 | 0.9 | - | - | - |
| Lactosucrose | - | - | 1.0 | - | - | - | - | - | - | - | - | 0.9 | - | - |
| Lactolin | - | - | - | 1.0 | - | - | - | - | - | - | - | - | 0.9 | - |
| Betaglucan | - | - | - | - | 1.0 | - | - | - | - | - | - | - | - | 0.9 |
| β-Sitosterol | - | - | - | - | - | 1.0 | - | - | - | 0.2 | - | - | - | - |
| β-Sitostanol | - | - | - | - | - | - | 1.0 | - | - | - | 0.1 | 0.1 | - | - |
| β-Sitosterol-E. | - | - | - | - | - | - | - | 1.0 | - | - | - | - | 0.1 | - |
| β-Sitostanol-E. | - | - | - | - | - | - | - | - | 1.0 | - | - | - | - | 0.1 |
| Bacterial numbers (CFU/ml) | 1.0 x 10⁶ | 1.5 x 10⁷ | 1.1 x 10⁷ | 1.6 x 10⁷ | 1.2 x 10⁷ | 3.3 x 10⁶ | 2.4 x 10⁶ | 2.7 x 10⁶ | 3.2 x 10⁶ | 4.1 x 10⁷ | 4.1 x 10⁷ | 4.1 x 10⁷ | 4.1 x 10⁷ | 4.4 x 10⁷ |
| β-Sitosterot-E. = β-Sitosterolpalmitate; β-Sitostanol-E. = β-Sitostanolstearate | | | | | | | | | | | | | | |

Starting from a control of 1.0 10⁶ CFU/ml (0) the addition of 1 % b.w. of various prebiotics (Comparative Examples C1-C4) increases the CFU by a factor of 10, while the addition of the sterols does only have a weak effect on the stimulation of cell growth (Comparative Examples C5-C8). Adding however mixture of prebiotics and sterols to the samples, the CFU numbers were multiplied by a factor of about 40 (Inventive Examples 1 to 5). The highest synergistic effect can be seen at a relation prebiotic : sterol of about 90 : 10.

**Table 2**

| **Effect of 1 % prebiotic, sterol and prebiotic/sterol mixture on** ***Lactobacterium*** | | | | | | | | | | | | | | |
|---|---|---|---|---|---|---|---|---|---|---|---|---|---|---|
| | **0** | **C9** | **C10** | **C11** | **C12** | **C13** | **C14** | **C15** | **C16** | **6** | **7** | **8** | **9** | **10** |
| Inulin | - | 1.0 | - | - | - | - | - | - | - | 0.8 | 0.9 | - | - | - |
| Lactosucrose | - | - | 1.0 | - | - | - | - | - | - | - | - | 0.9 | - | - |
| Lactolin | - | - | - | 1.0 | - | - | - | - | - | - | - | - | 0.9 | - |
| Betaglucan | - | - | - | - | 1.0 | - | - | - | - | - | - | - | - | 0.9 |
| β-Sitosterol | - | - | - | - | - | 1.0 | - | - | - | 0.2 | - | - | - | - |
| β-Sitostanol | - | - | - | - | - | - | 1.0 | - | - | - | 0.1 | 0.1 | - | - |
| β-Sitosterol-E. | - | - | - | - | - | - | - | 1.0 | - | - | - | - | 0.1 | - |
| β-Sitostanol-E. | - | - | - | - | - | - | - | - | 1.0 | - | - | - | - | 0.1 |
| Bacterial numbers (CFU/ml) | 2.8 x 10⁵ | 1.4 x 10⁶ | 1.1 x 10⁶ | 1.5 x 10⁶ | 1.1 x 10⁶ | 4.4 x 10⁵ | 4.4 x 10⁵ | 4.4 x 10⁵ | 4.5 x 10⁵ | 6.1 x 10⁶ | 6.2 x 10⁶ | 6.2 x 10⁶ | 6.2 x 10⁶ | 6.7 x 10⁶ |
| β-Sitosterol-E. = β-Sitosterolpalmitate; β-Sitostanol-E. = β-Sitostanotstearate | | | | | | | | | | | | | | |

Starting from a control of 2.8 10⁵ CFU/ml (0) the addition of 1 % b.w. of various prebiotics (Comparative Examples C9-C12) increases the CFU by a factor of 4, while the addition of the sterols does only have a weak effect on the stimulation of cell growth (Comparative Examples C13-C16). Adding however, mixture of prebiotics and sterols to the samples, the CFU numbers were multiplied by a factor of about 15 (Inventive Examples 6 to 10). The highest synergistic effect can be seen again at a relation prebiotic : sterol of about 90 : 10.

### Example 11

### Yoghurt composition

Soy milk is added to 15-75 parts by volume of cow milk to make 100 parts of the mixture. The mixture is then pasteurised at about 90 °C. for 15 seconds and then cooled. The cooled, pasteurised mixtures are then inoculated with 3 to 5 percent by volume of a yoghurt culture having 1:1 ratio of *Lactobacillus bulgaricus* and *Bifidobacterium adolescentis*. The incubation is carried out at about 42 °C. In about 2 hours thickening will occur. The fermentation is carried out for about 5.5 hours. The yoghurt compositions thus obtained is treated with 1 % - calculated on the amount of micro-organisms being present - of a 9:1 mixture of inulin and β-sitosterol. The products firm consistency and have a flavour like or substantially indistinguishable from that of a corresponding yoghurt composition using 100 percent of fresh cow milk. A small amount of citric acid can be added to the fermentation mixture to enhance the flavour of the final yoghurt composition. A suitable amount of citric acid is 0.5 percent based on the weight of the composition.

## Claims

1. Oral and/or topical compositions, comprising
(a) prebiotics and
(b) sterols or their esters.

2. Compositions according to claim 1, **characterised in that** said prebiotics (component a) are selected from the group consisting of fructooligosaccharides, inulins, isomaltooligosaccharides, lactilol, lactosucrose, Lactulose, Pyrodextrins, soy oligosaccharides, transgalactooligosaccharides, xylooligosaccharides and beta glucans.

3. Compositions according to claims 1 and/or 2, **characterised in that** said sterols or sterol esters (component b) represent phytosterols or phytosterol esters.

4. Compositions according to any of the claims 1 to 3, **characterised in that** component (b) represents sitosterol or sitostanol.

5. Compositions according to any of the claims 1 to 4, **characterised in that** component (b) represents esters of sterols or stanols with saturated or unsaturated fatty acids having 6 to 26 carbon atoms and up to 6 double bonds.

6. Compositions according to any of the claims 1 to 5, **characterised in that** they comprise component (a) and (b) in weight ratios of from 99 : 1 to 50 : 50.

7. Compositions according to any of the claims 1 to 5, **characterised in that** said mixtures are used in amounts of up to 10 % b.w. calculated on the micro-organisms being present in the final food composition.

8. Compositions according to any of the claims 1 to 6, **characterised in that** said mixtures are macro- or micro-encapsulated.

9. Food compositions, comprising
(a) prebiotics and
(b) sterols or their esters.

10. Use of mixtures, comprising
(a) prebiotics and
(b) sterols or their esters
for stimulating the growth of healthy bacteria.
